# EUROPEAN PATENT APPLICATION

(11) **EP 4 740 753 A1**
(43) Date of publication of application: **13.05.2026**
(21) Application number: 24839691.3
(22) Date of filing: 05.07.2024
(51) Int. Cl.: A23L 33/12, A23K 20/158, A61K 31/201, A61P 1/10

(54) **INTESTINAL REGULATOR**

(30) Priority: 07.07.2023 JP 2023112428
(71) Applicant: Noster Inc., Muko-shi, Kyoto 617-0006 (JP)
(72) Inventor: KITAO, Kohey, Muko-shi, Kyoto 617-0006 (JP)
(74) Representative: Hoffmann Eitle
(86) International application number: PCT/JP2024/024489
(87) International publication number: WO 2025/013799

(57) **Abstract**

The present invention provides an intestinal regulating agent containing a hydroxy fatty acid and/or an oxo fatty acid having 14 to 22 carbon atoms.

## Description

### Technical Field

The present invention relates to an intestinal regulating agent.

### Background Art

In recent years, various lifestyle diseases have become a problem due to changes in dietary habits, unbalanced diets, stress, and the like. One of these diseases is intestinal discomfort, such as constipation and diarrhea. Constipation occurs, for example, when stool remains in the intestines for a long time. Severe constipation can lead to an increase in harmful bacteria in the intestines, an increase in absorption of toxic substances and the like, and can potentially be a contributing factor to colorectal cancer. Therefore, the development of intestinal regulating agents using, as an active ingredient, a substance having an intestinal regulating effect for improving intestinal discomfort is underway.

In addition, it has been reported that some of hydroxy fatty acids and oxo fatty acids possess activity for improving lifestyle diseases, such as improving lipid metabolism (Patent Literature 1, and Non-Patent Literatures 1 and 2), and therefore, attention is now focusing on the physiological functions of hydroxy fatty acids and oxo fatty acids.

### Citation List

### Patent Literature

Patent Literature 1: Japanese Patent Laid-Open No. 2011-184411

### Non-Patent Literature

Non-Patent Literature 1: Kim Y-I,(2011), Mol. Nutr. Food Res., vol.55, p.585-593
Non-Patent Literature 2: Kim Y-I,(2012), PLoS ONE, vol.7, no.2, e31317

### Summary of Invention

### Technical Problem

However, it has not been previously known that a hydroxy fatty acid and/or an oxo fatty acid having 14 to 22 carbon atoms possess an intestinal regulating effect.

Therefore, an object of the present invention is to provide an intestinal regulating agent containing a hydroxy fatty acid and/or an oxo fatty acid having 14 to 22 carbon atoms.

### Solution to Problem

As a result of earnest studies, the present inventors have discovered that a hydroxy fatty acid and/or an oxo fatty acid having 14 to 22 carbon atoms possess an intestinal regulating effect, and thus, the present invention has been accomplished.

Specifically, the present invention provides the following.
[1] An intestinal regulating agent containing a hydroxy fatty acid and/or an oxo fatty acid having 14 to 22 carbon atoms.
[2] The intestinal regulating agent according to [1], wherein the hydroxy fatty acid has a hydroxyl group at positions 10, 11, 12, 13, 14, and/or 15, and the oxo fatty acid has a carbonyl group at positions 10, 11, 12, 13, 14, and/or 15.
[3] The intestinal regulating agent according to [1] or [2], wherein the hydroxy fatty acid or the oxo fatty acid is a saturated fatty acid, or an unsaturated fatty acid having one to five double bonds.
[4] The intestinal regulating agent according to any one of [1] to [3], wherein the hydroxy fatty acid is 10-hydroxy-cis-12-octadecenoic acid.
[5] The intestinal regulating agent according to any one of [1] to [4], wherein the intestinal regulating agent is a food product or a food additive.
[6] The intestinal regulating agent according to any one of [1] to [4], wherein the intestinal regulating agent is a medicament or a quasi-drug.
[7] The intestinal regulating agent according to any one of [1] to [4], wherein the intestinal regulating agent is feed or feed additive.
[8] The intestinal regulating agent according to any one of [1] to [4] for use for improving an intestinal environment.

The present invention also encompasses the following aspects.
[A1] A method for regulating an intestinal environment, comprising administering a physiologically effective amount of a hydroxy fatty acid and/or an oxo fatty acid having 14 to 22 carbon atoms to a subject in need thereof.
[A2] The method according to [A1], wherein the hydroxy fatty acid has a hydroxyl group at positions 10, 11, 12, 13, 14, and/or 15, and the oxo fatty acid has a carbonyl group at positions 10, 11, 12, 13, 14, and/or 15.
[A3] The method according to [A1] or [A2], wherein the hydroxy fatty acid or the oxo fatty acid is a saturated fatty acid, or an unsaturated fatty acid having 1 to 5 double bonds.
[A4] The method according to any one of [A1] to [A3], wherein the hydroxy fatty acid is 10-hydroxy-cis-12-octadecenoic acid.

In the methods of [A1] to [A4], the intestinal regulating agent according to any one of [1] to [8] may be administered, or a composition according to any one of [E1] to [E7] described below may be administered.

[B1] A hydroxy fatty acid and/or an oxo fatty acid having 14 to 22 carbon atoms for use in a method for regulating an intestinal environment.
[B2] The fatty acid according to [B1], wherein the hydroxy fatty acid has a hydroxyl group at positions 10, 11, 12, 13, 14, and/or 15, and the oxo fatty acid has a carbonyl group at positions 10, 11, 12, 13, 14, and/or 15.
[B3] The fatty acid according to [B1] or [B2], wherein the hydroxy fatty acid or the oxo fatty acid is a saturated fatty acid, or an unsaturated fatty acid having 1 to 5 double bonds.
[B4] The fatty acid according to any one of [B1] to [B3], wherein the hydroxy fatty acid is 10-hydroxy-cis-12-octadecenoic acid.
[C1] Use of a hydroxy fatty acid and/or an oxo fatty acid having 14 to 22 carbon atoms for regulating an intestinal environment.
[C2] The use according to [C1], wherein the hydroxy fatty acid has a hydroxyl group at positions 10, 11, 12, 13, 14, and/or 15, and the oxo fatty acid has a carbonyl group at positions 10, 11, 12, 13, 14, and/or 15.
[C3] The use according to [C1] or [C2], wherein the hydroxy fatty acid or the oxo fatty acid is a saturated fatty acid, or an unsaturated fatty acid having 1 to 5 double bonds.
[C4] The use according to any one of [C1] to [C3], wherein the hydroxy fatty acid is 10-hydroxy-cis-12-octadecenoic acid.
[D1] Use of a hydroxy fatty acid and/or an oxo fatty acid having 14 to 22 carbon atoms for manufacturing a composition or an intestinal regulating agent for regulating an intestinal environment.
[D2] The use according to [D1], wherein the hydroxy fatty acid has a hydroxyl group at positions 10, 11, 12, 13, 14, and/or 15, and the oxo fatty acid has a carbonyl group at positions 10, 11, 12, 13, 14, and/or 15.
[D3] The use according to [D1] or [D2], wherein the hydroxy fatty acid or the oxo fatty acid is a saturated fatty acid, or an unsaturated fatty acid having 1 to 5 double bonds.
[D4] The use according to any one of [D1] to [D3], wherein the hydroxy fatty acid is 10-hydroxy-cis-12-octadecenoic acid.
[D5] The use according to any one of [D1] to [D4], wherein the composition is a food product.
[D6] The use according to any one of [D1] to [D4], wherein the composition is a medicament or a quasi-drug.
[D7] The use according to any one of [D1] to [D4], wherein the composition is feed.
[E1] A composition for regulating an intestinal environment, containing a hydroxy fatty acid and/or an oxo fatty acid having 14 to 22 carbon atoms.
[E2] The composition according to [E1], wherein the hydroxy fatty acid has a hydroxyl group at positions 10, 11, 12, 13, 14, and/or 15, and the oxo fatty acid has a carbonyl group at positions 10, 11, 12, 13, 14, and/or 15.
[E3] The composition according to [E1] or [E2], wherein the hydroxy fatty acid or the oxo fatty acid is a saturated fatty acid, or an unsaturated fatty acid having 1 to 5 double bonds.
[E4] The composition according to any one of [E1] to [E3], wherein the hydroxy fatty acid is 10-hydroxy-cis-12-octadecenoic acid.
[E5] The composition according to any one of [E1] to [E4], wherein the composition is a food product.
[E6] The composition according to any one of [E1] to [E4], wherein the composition is a medicament or a quasi-drug.
[E7] The composition according to any one of [E1] to [E4], wherein the composition is feed.

### Advantageous Effect of Invention

The present invention can provide an intestinal regulating agent containing a hydroxy fatty acid and/or an oxo fatty acid having 14 to 22 carbon atoms.

### Description of Embodiments

Now, embodiments for practicing the present invention will be described in detail. It is noted that the present invention is not limited to the following embodiments, but can be implemented with various modifications made within the spirit and the scope thereof.

### (Intestinal Regulating Effect)

An intestinal regulating agent of the present embodiment has an intestinal regulating effect. In the present embodiment, the intestinal regulating effect is not particularly limited, and includes, for example, a regulating effect on an intestinal environment, such as an improving effect on intestinal discomfort, and an improving effect on an intestinal environment. Specific examples of the effect are not particularly limited and include a bowel movement-promoting effect, a bowel movement-improving effect, a constipation-relieving effect, and a constipation-preventive effect. The intestinal regulating agent preferably has, for example, without particular limitation, a regulating effect on bowel movements, an increasing effect on the amount of stool, an increasing effect on the frequency of defecation, and a relieving effect on abdominal pain associated with constipation.

Without wishing to be bound by any specific theory, the present inventors presume as follows regarding why a hydroxy fatty acid and/or an oxo fatty acid having 14 to 22 carbon atoms possess the intestinal regulating effect. It is thought that a hydroxy fatty acid and/or an oxo fatty acid having 14 to 22 carbon atoms have a promoting effect on the secretion of incretins, that is, gastrointestinal hormones (e.g., GLP-1) via a long-chain fatty acid receptor in the intestinal tract (e.g., GPR40 and GPR120). It is thought that these fatty acids have an improving effect on the gut microbiota and a promoting effect on intestinal peristalsis via a prostanoid receptor (e.g., EP3). In particular, it is thought that when the fatty acid has 14 to 22 carbon atoms and has a hydroxyl group and/or a carboxyl group, the affinity thereof with a long-chain fatty acid receptor, a prostanoid receptor, and the like is enhanced.

The intestinal regulating agent of the present embodiment may be applied also for improving an intestinal environment. The application for improving an intestinal environment includes, for example, without particular limitation, changing the gut microbiota, such as increasing bacteria belonging to the phyla Firmicutes, Proteobacteria, Bacteroidetes, Actinomycetota, and Euryarchaeota.

The intestinal regulating agent of the present embodiment is expected to improve, alleviate, treat, and prevent intestinal dysfunctions such as constipation, diarrhea, and defecation disorders owing to the intestinal regulating effect thereof. The intestinal regulating agent of the present embodiment may be a bowel movement promoting agent, a bowel movement improving agent, a constipation improving agent, an intestinal environment improving agent, or the like.

Herein, the description of the intestinal regulating agent is understood to be replaced by description of a composition for regulating an intestinal environment.

### (Hydroxy Fatty Acid and Oxo Fatty Acid)

Herein, a fatty acid means a compound having a structure having a carboxyl group (a group represented by COOH, which may be in a salt form) at one end of a hydrocarbon chain. A hydroxy fatty acid means a fatty acid having one or more hydroxyl groups that are different from the terminal carboxyl group, and an oxo fatty acid means a fatty acid having one or more carbonyl groups that are different from the terminal carboxyl group.

In the present embodiment, the hydroxy fatty acid or the oxo fatty acid has 14 to 22 carbon atoms, and is, without particular limitation, preferably a hydroxy fatty acid or an oxo fatty acid having 14, 16, 18, 20, or 22 carbon atoms, and more preferably a hydroxy fatty acid or an oxo fatty acid having 18 carbon atoms.

In the present embodiment, the hydroxy fatty acid or the oxo fatty acid may have a straight-chain, branched, or cyclic hydrocarbon chain, and without particular limitation, preferably has a straight-chain hydrocarbon chain.

In the present embodiment, without particular limitation, the hydroxy fatty acid has a hydroxyl group preferably at positions 10, 11, 12, 13, 14, and/or 15, has a hydroxyl group more preferably at positions 10, 12, and/or 13, and has a hydroxyl group particularly preferably at position 10. Without particular limitation, the oxo fatty acid has a carbonyl group preferably at positions 10, 11, 12, 13, 14, and/or 15, has a carbonyl group more preferably at positions 10, 12, and/or 13, and has a carbonyl group particularly preferably at position 10. The number of hydroxyl groups or carbonyl groups may be one or more and is not particularly limited, and is preferably one to five, more preferably one to three, and particularly preferably one. In addition, the hydroxy fatty acid may further have a carbonyl group, and the oxo fatty acid may further have a hydroxyl group.

In the present embodiment, without particular limitation, a hydroxy fatty acid having 14 or 16 carbon atoms has a hydroxyl group preferably at position 10, and an oxo fatty acid having 14 or 16 carbon atoms has a carbonyl group preferably at position 10.

Without particular limitation, a hydroxy fatty acid having 18 carbon atoms has a hydroxyl group preferably at positions 10, 12 and/or 13, and an oxo fatty acid having 18 carbon atoms has a carbonyl group preferably at positions 10, 12, and/or 13.

Without particular limitation, a hydroxy fatty acid having 20 carbon atoms has a hydroxyl group preferably at positions 12, and/or 15, and an oxo fatty acid having 20 carbon atoms has a carbonyl group preferably at positions 12, and/or 15.

Without particular limitation, a hydroxy fatty acid having 22 carbon atoms has a hydroxyl group preferably at positions 11, and/or 14, and an oxo fatty acid having 22 carbon atoms has a carbonyl group preferably at positions 11, and/or 14.

In the present embodiment, without particular limitation, the hydroxy fatty acid or the oxo fatty acid is preferably a saturated fatty acid, or an unsaturated fatty acid having one to five double bonds, more preferably a saturated fatty acid, or an unsaturated fatty acid having one to three double bonds, and particularly preferably a saturated fatty acid, or an unsaturated fatty acid having one double bond.

When the fatty acid is an unsaturated fatty acid, without particular limitation, the unsaturated fatty acid preferably has one or more trans-type double bonds or cis-type double bonds at position 4 to position 19, and more preferably has one or more trans-type double bonds or cis-type double bonds at positions 4, 5, 6, 7, 8, 9, 11, 12, 13, 14, 15, 17, and/or 19.

In the present embodiment, without particular limitation, a hydroxy fatty acid having 14 or 16 carbon atoms is preferably a saturated fatty acid.

Without particular limitation, a hydroxy fatty acid or an oxo fatty acid having 18 carbon atoms is preferably a saturated fatty acid, or has one or more trans-type double bonds or cis-type double bonds at positions 6, 9, 11, 12, and/or 15, and is more preferably a saturated fatty acid, or has one or more cis-type double bonds at positions 6, 9, and/or 15.

Without particular limitation, a hydroxy fatty acid or an oxo fatty acid having 20 carbon atoms is preferably a saturated fatty acid, or has one or more trans-type double bonds or cis-type double bonds at positions 5, 8, 11, 14, and/or 17, and is more preferably a saturated fatty acid, or has one or more cis-type double bonds at positions 5, 8, and/or 17.

Without particular limitation, a hydroxy fatty acid or an oxo fatty acid having 22 carbon atoms is preferably a saturated fatty acid, or has one or more cis-type double bonds at positions 4, 7, 10, 13, 16, and/or 19, and is more preferably a saturated fatty acid, or has one or more cis-type double bonds at positions 4, 7, 16, and/or 19.

In the present embodiment, hydroxy fatty acids having 14 and 16 carbon atoms and having a hydroxyl group at position 10 is not particularly limited, and examples include 10-hydroxy-tetradecanoic acid and 10-hydroxy-hexadecanoic acid. Oxo fatty acids having 14 and 16 carbon atoms and having a carbonyl group at position 10 is not particularly limited, and examples include 10-oxo-tetradecanoic acid and 10-oxo-hexadecanoic acid.

In the present embodiment, a hydroxy fatty acid having 18 carbon atoms and having a hydroxyl group at position 10 is not particularly limited, and examples include 10-hydroxy-cis-12-octadecenoic acid, 10-hydroxy-cis-12,cis-15-octadecadienoic acid, 10-hydroxy-cis-6,cis-12,cis-15-octadecatrienoic acid, 10-hydroxy-cis-6,cis-12-octadecadienoic acid, 10-hydroxyoctadecanoic acid, 10-hydroxy-cis-15-octadecenoic acid, 10-hydroxy-cis-6,cis-15-octadecadienoic acid, 10-hydroxy-cis-6-octadecenoic acid, 10-hydroxy-trans-11-octadecenoic acid, 10-hydroxy-trans-11,cis-15-octadecadienoic acid, 10-hydroxy-cis-6,trans-11,cis-15-octadecatrienoic acid, 10-hydroxy-cis-6,trans-11-octadecadienoic acid, 10,13-dihydroxyoctadecanoic acid, 10,13-dihydroxy-cis-15-octadecenoic acid, 10,13-dihydroxy-cis-6-octadecenoic acid, 10,12-dihydroxyoctadecanoic acid, and 10,13-dihydroxy-cis-6,cis-15-octadecadienoic acid.

In the present embodiment, an oxo fatty acid having 18 carbon atoms and having a carbonyl group at position 10 is not particularly limited, and examples include 10-oxo-cis-12-octadecenoic acid, 10-oxo-cis-12,cis-15-octadecadienoic acid, 10-oxo-cis-6,cis-12,cis-15-octadecatrienoic acid, 10-oxo-cis-6,cis-12-octadecadienoic acid, 10-oxooctadecanoic acid, 10-oxo-cis-15-octadecenoic acid, 10-oxo-cis-6,cis-15-octadecadienoic acid, 10-oxo-cis-6-octadecenoic acid, 10-oxo-trans-11-octadecenoic acid, 10-oxo-trans-11,cis-15-octadecadienoic acid, 10-oxo-cis-6,trans-11,cis-15-octadecatrienoic acid, 10-oxo-cis-6,trans-11-octadecadienoic acid, 10,13-dioxo-octadecanoic acid, 10,13-dioxo-cis-6-octadecenoic acid, 10,13-dioxo-cis-15-octadecenoic acid, and 10,13-dioxo-cis-6,cis-15-octadecadienoic acid.

In the present embodiment, a hydroxy fatty acid having 18 carbon atoms and having a hydroxyl group at position 12 is not particularly limited, and examples include 12-hydroxy-cis-9-octadecenoic acid, and 12-hydroxyoctadecanoic acid.

In the present embodiment, an oxo fatty acid having 18 carbon atoms and having a carbonyl group at position 12 is not particularly limited, and examples include 12-oxo-cis-9-octadecenoic acid, and 12-oxo-octadecanoic acid.

In the present embodiment, a hydroxy fatty acid having 18 carbon atoms and having a hydroxyl group at position 13 is not particularly limited, and examples include 13-hydroxy-cis-9-octadecenoic acid, 13-hydroxy-cis-9,cis-15-octadecadienoic acid, 13-hydroxy-cis-6,cis-9-octadecadienoic acid, 13-hydroxy-cis-6,cis-9,cis-15-octadecatrienoic acid, 10-oxo-13-hydroxyoctadecanoic acid, 10-oxo-13-hydroxy-cis-6-octadecenoic acid, 10-oxo-13-hydroxy-cis-15-octadecenoic acid, 10-oxo-13-hydroxy-cis-6,cis-15-octadecadienoic acid, 13-hydroxy-cis-5,cis-9-octadecadienoic acid, and 13-hydroxy-trans-5,cis-9-octadecadienoic acid.

In the present embodiment, an oxo fatty acid having 18 carbon atoms and having a carbonyl group at position 13 is not particularly limited, and examples include 13-oxo-cis-9-octadecenoic acid, 13-oxo-cis-9,cis-15-octadecadienoic acid, 13-oxo-cis-6,cis-9-octadecadienoic acid, 13-oxo-cis-6,cis-9,cis-15-octadecatrienoic acid, 13-oxo-cis-5,cis-9-octadecadienoic acid, and 13-oxo-trans-5,cis-9-octadecadienoic acid.

In the present embodiment, a hydroxy fatty acid having 20 carbon atoms and having a hydroxyl group at position 12 is not particularly limited, and examples include 12-hydroxy-cis-14-eicosenoic acid, 12-hydroxy-cis-14,cis-17-eicosadienoic acid, 12-hydroxy-cis-8,cis-14-eicosadienoic acid, 12-hydroxy-cis-5,cis-8-eicosadienoic acid, 12-hydroxy-cis-8,cis-14,cis-17-eicosatrienoic acid, 12-hydroxy-cis-5,cis-8,cis-14-eicosatrienoic acid, and 12-hydroxy-cis-5,cis-8,cis-14,cis-17-eicosatetraenoic acid.

In the present embodiment, an oxo fatty acid having 20 carbon atoms and having a carbonyl group at position 12 is not particularly limited, and examples include 12-oxo-cis-14-eicosenoic acid, 12-oxo-cis-14,cis-17-eicosadienoic acid, 12-oxo-cis-8,cis-14-eicosadienoic acid, 12-oxo-cis-5,cis-8-eicosadienoic acid, 12-oxo-cis-8,cis-14,cis-17-eicosatrienoic acid, 12-oxo-cis-5,cis-8,cis-14-eicosatrienoic acid, and 12-oxo-cis-5,cis-8,cis-14,cis-17-eicosatetraenoic acid.

In the present embodiment, a hydroxy fatty acid having 20 carbon atoms and having a hydroxyl group at position 15 is not particularly limited, and examples include 15-hydroxy-cis-11-eicosenoic acid, 15-hydroxy-cis-11,cis-17-eicosadienoic acid, 15-hydroxy-cis-8,cis-11-eicosadienoic acid, 15-hydroxy-cis-5,cis-8,cis-11-eicosatrienoic acid, 15-hydroxy-cis-8,cis-11,cis-17-eicosatrienoic acid, 15-hydroxy-cis-5,cis-11-eicosadienoic acid, and 15-hydroxy-cis-5,cis-11,cis-17-eicosatrienoic acid.

In the present embodiment, an oxo fatty acid having 20 carbon atoms and having a carbonyl group at position 15 is not particularly limited, and examples include 15-oxo-cis-11-eicosenoic acid, 15-oxo-cis-11,cis-17-eicosadienoic acid, 15-oxo-cis-8,cis-11-eicosadienoic acid, 15-oxo-cis-5,cis-8,cis-11-eicosatrienoic acid, 15-oxo-cis-8,cis-11,cis-17-eicosatrienoic acid, 15-oxo-cis-5,cis-11-eicosadienoic acid, and 15-oxo-cis-5,cis-11,cis-17-eicosatrienoic acid.

In the present embodiment, a hydroxy fatty acid having 22 carbon atoms and having a hydroxyl group at position 11 is not particularly limited, and examples include 11-hydroxy-cis-7,cis-13,cis-16,cis-19-docosatetraenoic acid, and 11-hydroxy-cis-4,cis-7,cis-13,cis-16,cis-19-docosapentaenoic acid.

In the present embodiment, an oxo fatty acid having 22 carbon atoms and having a carbonyl group at position 11 is not particularly limited, and examples include 11-oxo-cis-7,cis-13,cis-16,cis-19-docosatetraenoic acid, and 11-oxo-cis-4,cis-7,cis-13,cis-16,cis-19-docosapentaenoic acid.

In the present embodiment, a hydroxy fatty acid having 22 carbon atoms and having a hydroxyl group at position 14 is not particularly limited, and an example includes 14-hydroxy-cis-4,cis-7,cis-10,cis-16,cis-19-docosapentaenoic acid.

In the present embodiment, an oxo fatty acid having 22 carbon atoms and having a carbonyl group at position 11 is not particularly limited, and an example includes 14-oxo-cis-4,cis-7,cis-10,cis-16,cis-19-docosapentaenoic acid.

In the present embodiment, the hydroxy fatty acid is not particularly limited, and from the viewpoint of more effectively and definitely achieving the effects and advantages of the present invention, is preferably a hydroxy fatty acid having 18 carbon atoms and having a hydroxyl group at position 10, more preferably a hydroxy fatty acid having 18 carbon atoms and having one or more cis-type double bonds at position 12, and particularly preferably 10-hydroxy-cis-12-octadecenoic acid.

### (Method for Producing Hydroxy Fatty Acid/Oxo Fatty Acid)

In the present embodiment, the hydroxy fatty acid and/or the oxo fatty acid having 14 to 22 carbon atoms can be produced by any known method. Without particular limitation, the fatty acid can be produced by methods described in, for example, WO2013/168310, WO2015/111699, WO2020/203751, Biochemical and Biophysical Research Communications 416 (2011), p. 188-193, and the like.

### (Intestinal Regulating Agent)

Without particular limitation, the intestinal regulating agent of the present embodiment may be used, for example, as a food product or a food additive, a medicament or a quasi-drug, or feed or feed additive.

One type of hydroxyl fatty acid and/or oxo fatty acid may be compounded in the intestinal regulating agent, or two or more types may be used in combination.

Without particular limitation, the intestinal regulating agent of the present embodiment may be used for a human, or used for a pet or livestock.

In the present embodiment, the dosage or intake of the intestinal regulating agent is not particularly limited, and may be determined depending on, for example, the age and weight of a patient or a recipient, symptoms, administration time, dosage form, administration method, drug combination, and the like.

When the intestinal regulating agent is orally administered as a medicament, the dosage is, in terms of the total amount of the active ingredient, that is, the hydroxy fatty acid and/or the oxo fatty acid, not particularly limited, and is preferably 0.02 to 100 mg/kg body weight per day for an adult, and more preferably 0.2 to 50 mg/kg body weight. When the intestinal regulating agent is administered parenterally, the dosage is not particularly limited, and is preferably 0.002 mg to 50 mg/kg body weight per day for an adult, and more preferably 0.02 to 50 mg/kg body weight. The dosage may be administered once a day or divided into several doses (2 to 5 times).

When the intestinal regulating agent is ingested as a food product, the intake, in terms of the total intake of the active ingredient, that is, the hydroxylated fatty acid and/or the oxo fatty acid, is preferably 1 to 6000 mg per day for an adult, more preferably 10 to 3000 mg, and particularly preferably 100 mg to 1500 mg.

When the intestinal regulating agent is ingested as feed, the intake of the feed is not particularly limited, and may be determined in accordance with the intake of a food product and the dosage of a medicament.

### (Food Product/Food Additive)

The intestinal regulating agent of the present embodiment can be used as a food product or a food additive. The food product is not particularly limited, and examples include general food products, health food products, functional food products, nutritional supplements, food with health claims (e.g., food for specified health uses, food with functional claims, and food with nutrient function claims), and food for special dietary uses (e.g., infant food, maternity food, and medical food).

The intestinal regulating agent of the present embodiment may be provided and sold as a food product with labeling indicating the use for regulating an intestinal environment, improving intestinal discomfort, improving an intestinal environment, and the like.

Such a "labeling" act includes all acts intended to inform consumers of the aforementioned uses, and any expression that can lead consumers to recall or think metaphorically of these uses falls under the "labeling" act of the present embodiment, regardless of the purpose of the labeling, the content of the labeling, or the object/medium of the labeling.

The labeling is not particularly limited, and is preferably labeling that appeals for regulating an intestinal environment, improving intestinal discomfort, and improving an intestinal environment, and more preferably is labeling for a food product that has been permitted or notified as necessary (i.e., food with health claims such as food for specified health uses, food with functional claims, and food with nutrient function claims, and food for special dietary uses). Examples of the labeling include: "This product has the function of improving bowel movements/defection," "This product has the function of regulating the abdominal condition for those concerned about bowel regularity," and "this product is a food product suitable for those who wish to regulate their abdominal condition or are concerned about bowel regularity." Food products for which functional labeling has been permitted or notified can be distinguished from general food products.

The form of the food product or the food additive is not particularly limited, and examples include orally ingestible forms such as a solution, a suspension, a powder, and a solid molded product. Such forms are not particularly limited, and examples include supplements (e.g., a powder, a granule, a soft capsule, a hard capsule, a tablet, a chewable tablet, an orally disintegrating tablet, a syrup, and a liquid), beverages (e.g., a carbonated drink, a lactic acid drink, a sports drink, a fruit juice drink, a vegetable drink, a soy milk drink, a coffee drink, a tea drink, a powdered drink, a concentrated drink, a nutritional drink, and an alcoholic drink), sweets (e.g., a gummy, a jelly, a chewing gum, a chocolate, a cooky, a candy, a caramel, Japanese sweet, and snack food), instant foods (e.g., instant noodles, retort food, canned food, microwave food, an instant soup/miso soup, and freeze-dried food), oils, oil and fat foods (e.g., mayonnaise, dressing, butter, cream, and margarine), flour products (e.g., bread, pasta, noodles, a cake mix, and bread crumbs); seasonings (e.g., a sauce, a processed tomato seasoning, a flavor seasoning, a cooking mix, and a soup base); and processed livestock products (e.g., a ham/sausage).

Without particular limitation, in the food product, for example, various nutrients, various vitamins (e.g., Vitamin A, Vitamin B1, Vitamin B2, Vitamin B6, Vitamin C, Vitamin D, Vitamin E, and Vitamin K), various minerals (e.g., magnesium, zinc, iron, sodium, potassium, and selenium), dietary fiber, a dispersing agent, a stabilizer such as an emulsifier, a sweetener, a flavor component (e.g., citric acid and malic acid), a flavor, royal jelly, propolis, agaricus and the like may be blended. As long as the effects of the present invention are not impaired, another fatty acid may be contained in addition to the hydroxy fatty acid and the oxo fatty acid having 14 to 22 carbon atoms.

### (Medicament)

The intestinal regulating agent of the present embodiment can be used as a medicament, or a quasi-drug. The dosage form of the medicament or the quasi-drug is not particularly limited, and examples include a powder, a granule, a pill, a soft capsule, a hard capsule, a tablet, a chewable tablet, an orally disintegrating tablet, a syrup, a liquid, a suspension, a suppository, an ointment, a cream, a gel, a patch, an inhalant, and an injection. Furthermore, the medicament or the quasi-drug may be administered orally or parenterally.

In the preparation of a formulation, the medicament or the quasi-drug of a desired dosage form can be produced by any usual method. However, in the present embodiment, when the hydroxy fatty acid or the oxo fatty acid is poorly soluble in water, it may be dissolved in a non-hydrophilic organic solvent such as a vegetable oil or animal oil, or may be dispersed or emulsified in an aqueous solution using a homogenizer (high-pressure homogenizer) together with an emulsifier, a dispersing agent, a surfactant, or the like.

In the preparation of the formulation, an additive may be used in addition to the active ingredient. The additive is not particularly limited, and examples include animal and vegetable oils such as soybean oil, safflower oil, olive oil, germ oil, sunflower oil, beef tallow, and sardine oil, polyhydric alcohols such as polyethylene glycol, propylene glycol, glycerin, and sorbitol, surfactants such as sorbitan fatty acid esters, sucrose fatty acid esters, glycerin fatty acid esters, and polyglycerin fatty acid esters, excipients such as purified water, lactose, starch, crystalline cellulose, D-mannitol, lecithin, gum arabic, sorbitol solution, and sugar solution, and a sweetener, a coloring agent, a pH adjuster, a fragrance, a binder, a disintegrating agent, a stabilizing agent, and a lubricating agent. Furthermore, a liquid formulation may be in a form that is dissolved or suspended in water or another suitable medium at the time of dosing. A tablet and a granule may be coated by a known method. As long as the effects of the present invention are not impaired, another fatty acid may be contained in addition to the hydroxy fatty acid and the oxo fatty acid having 14 to 22 carbon atoms.

### (Feed and Feed Additive)

The intestinal regulating agent of the present embodiment can be used as feed or feed additive. The feed is not particularly limited, and examples include pet food, and livestock or aquaculture feed additives.

### Examples

The present embodiment will now be more specifically described by way of Examples and Comparative Examples, and it is noted that the present embodiment is not limited only to these examples.

The influence of ingesting a hydroxy fatty acid and/or an oxo fatty acid having 14 to 22 carbon atoms on human bowel movements and intestinal environment was investigated.

### [Test Method]

### 1. Subject Selection Criteria

Subjects selected were Japanese individuals, both male and female, who were adults, healthy, had a bowel movement frequency of 3 to 5 times per week, and had a relatively low bowel movement frequency in one week before a screening and pre-ingestion test.

### 2. Test Food Product

Test Food Group: capsule containing 10-hydroxy-cis-12-octadecenoic acid (hereinafter also referred to as "HYA") (100 mg), capsule shell (gelatin, glycerin, caramel) (130 mg), other reaction by-products, etc. (100 mg) (prepared from safflower oil-derived fatty acid through a reaction described in WO2013/168310)
Placebo Group: placebo capsule containing capsule shell (gelatin, glycerin, caramel) (130 mg)

### 3. Test Period, and Dosage and Administration

### Intervention Period: two weeks

Test Food Group: Three capsules (300 mg in terms of HYA) were ingested three times per day with water or lukewarm water before each meal.

Placebo Group: Three capsules were ingested three times per day with water or lukewarm water before each meal (the daily dose was ingested within that day. If forgotten, the dose was ingested as soon as the subject remembered).

### 4. Measurement Items

### (1) Bowel Movement Diary

- Details: The test participants themselves recorded the presence or absence of bowel movements and their condition in a bowel movement diary.
- Survey Items: frequency of bowel movements, frequency of defecation, amount of stool
- Evaluation method: For the frequency of bowel movements and the frequency of defecation, the frequencies recorded in the bowel movement dairy were evaluated. The amount of stool was evaluated based on the size of a film case-type container No. 7 (distributer: Roppon-Ashi Entomological Books, Chiyoda-ku, Tokyo). The recording was performed daily for one week from one week before the screening and pre-ingestion test (Scr) until the day before Scr, and for three weeks from one week before the start of the ingestion until the day before the final test. The 7 days before the Scr were defined as Period I, the 7 days before the start of the ingestion were defined as Period II, the 7 days after the start of the ingestion were defined as Period III, and a period from the 8th day of the ingestion until a previous day of a test after 2-week ingestion was defined as Period IV. The frequency of bowel movements, the frequency of defecation, and the amount of stool were evaluated as the total for every seven days. It is noted that Period IV was shortened or extended in some cases due to the circumstances of a test participant. A shortening of up to one day was permitted, and an extension of up to two days was permitted. If the period was extended, Period IV was defined as the 7 days immediately before the date of the test after 2-week ingestion. If the extension was three days or more, Period IV was defined as the 7 days immediately preceding the second day of the extension.

### (2) The Japanese Version of the Constipation Assessment Scale: CAS-MT

- Details: subjective symptom was evaluated using CAS-MT.
- Survey Items: question items of CAS-MT
- Evaluation Method: An answer to each question item was evaluated on a 3-point scale from 0 to 2 points. The evaluation was performed before the screening and pre-ingestion test and after the test after 2-week ingestion.

### (3) Stool Sample Management

- Details: Each test participant collected three stool samples in total, one stool sampling tube with preservation solution and two stool sampling tubes without preservation solution, and the stool samples were collected from the subject upon his/her visit to a clinic. The stool sampling tubes with preservation solution were stored under refrigeration, and the stool sampling tubes without preservation solution were stored under freezing, and these were then shipped to a testing laboratory.
   * The stool sample was collected as the first stool in a period from 3 days before the test day up to and including the test day, and the subject sent the stool sample by frozen delivery to the testing laboratory.
- Survey Item: bacterial occupancy rate in stool
- Evaluation Method: The evaluation was performed in the screening and pre-ingestion test and the test after 2-week ingestion.

### 5. Outcome

### (1) Efficacy Evaluation Items

i. Primary Outcome
   a. actual frequency of bowel movements in Period IV
ii. Secondary Outcome
   a. change in frequency of bowel movements in Period IV from that in Period I
   b. actual frequencies of bowel movements in Periods II and III, and change from that in Period I
   c. actual values of frequencies of defecation and amounts of stool in Periods II, III, and IV, and change from those in Period I
   d. proportion of each state for stool shape, stool odor, and feeling of relief in Periods II, III, and IV
   e. actual CAS scores after 2-week ingestion, and change from that in screening and pre-ingestion test
   f. actual values for each question item of CAS-MT after 2-week ingestion
   g. actual values for diversity index and bacterial occupancy rate in stool after 2-week ingestion
   h. change in diversity index and bacterial occupancy rate in stool after 2-week ingestion from those in the screening and pre-ingestion test

### 6. Sample Size

(1) Number of screened subjects: 73
(2) Target number of subjects: 52
(3) Number of treated subjects: 56
(4) Rationale for sample size

This test assumed a large difference in the actual frequency of bowel movements between the test food group and the placebo group after 2-week ingestion, and based on Cohen's suggestion, d = 0.80 was used. When the number of subjects was calculated with a statistical significance level (α) set to 0.05 and statistical power (1 - β) set to 0.80, the calculated number of subjects required was 52 (26 per group). Furthermore, anticipating dropouts and non-compliance with the protocol during the test, the number of treated subjects was increased to 56 (28 per group).

### [Test Results]

### 1. Explanation of Terminologies

**[Table 1]**

| Abbreviation | Formal Name |
|---|---|
| ITT | Intention to treat |
| SAF | Safety analysis population |
| VISIT1 / Scr | Before screening and ingestion (baseline) |
| VISIT2 | Period of test after 2-week ingestion |
| Period I | 7 days before Scr |
| Period II | 7 days before start of ingestion |
| Period III | 7 days after start of ingestion |
| Period IV | from 8th day of ingestion until previous day of test after 2-week ingestion |
| Change | Change in test after 2-week ingestion from baseline |
| Change 1 | Change in Period II from Period I |
| Change 2 | Change in Period III from Period I |
| Change 3 | Change in Period IV from Period I |
| Mean | Mean value |
| SD | Standard deviation |
| SE | Standard error |
| 95%CI-, 95%Cl+ | 95% confidence interval |
| Med | Median |
| Min | Minimum value |
| Max | Maximum value |
| Q1 | First quartile |
| Q3 | Third quartile |
| CAS-MT | The Japanese Version of the Constipation Assessment Scale [middle term] |

### 2. Interpretation of Numerical Values of Efficacy Evaluation Items

**[Table 2]**

| Evaluation Item | Direction of numerical change (↑,↓) | |
|---|---|---|
| | better | worse |
| Frequency of bowel movements | ↑ | ↓ |
| Frequency of defecation | ↑ | ↓ |
| Amount of stool | ↑ | ↓ |
| Questions of CAS-MT | ↓ | ↑ |

### 3. Primary Outcome and Secondary Outcome

### 3.1. Bowel Movement Diary

### 3.1.1. Frequency of Defecation, Frequency of Bowel Movements, and Amount of Stool

The Mean and SD, Med, Min, Max, a difference between groups (Δ) and its SE, 95% CI-, 95% Cl+, and statistical analysis results for the frequency of defecation, the frequency of bowel movements, and the amount of stool are shown in a table below. The following shows, regarding the items where a significant difference was observed, the time point at which the significant difference was observed, the Mean and SD, the difference between groups, and 95% Cl- and 95% Cl+ thereof, and the significance probability.

### (1) Frequency of Bowel Movements

Among time points where a significant difference was observed, time points at which the test food group showed higher values than the placebo group were Period IV (placebo group: 4.9 ± 2.1 times, test food group: 6.2 ± 2.0 times, difference between groups: 1.3 times [0.3, 2.4], P = 0.016) and Change 3 (placebo group: 0.9 ± 2.2 times, test food group: 2.2 ± 1.9 times, difference between groups: 1.3 times [0.3, 2.4], P = 0.016).

### (2) Frequency of Defecation

Among time points where a significant difference was observed, time points at which the test food group showed higher values than the placebo group were Period IV (placebo group: 4.5 ± 1.7 days, test food group: 5.2 ± 1.4 days, difference between groups: 0.8 days [0.0, 1.5], P = 0.045) and Change 3 (placebo group: 0.6 ± 1.7 days, test food group: 1.5 ± 1.4 days, difference between groups: 0.8 days [0.0, 1.5], P = 0.045).

From the above, it was found that the ingestion of the hydroxy fatty acid and/or the oxo fatty acid having 14 to 22 carbon atoms significantly increased the frequency of spontaneous bowel movements.

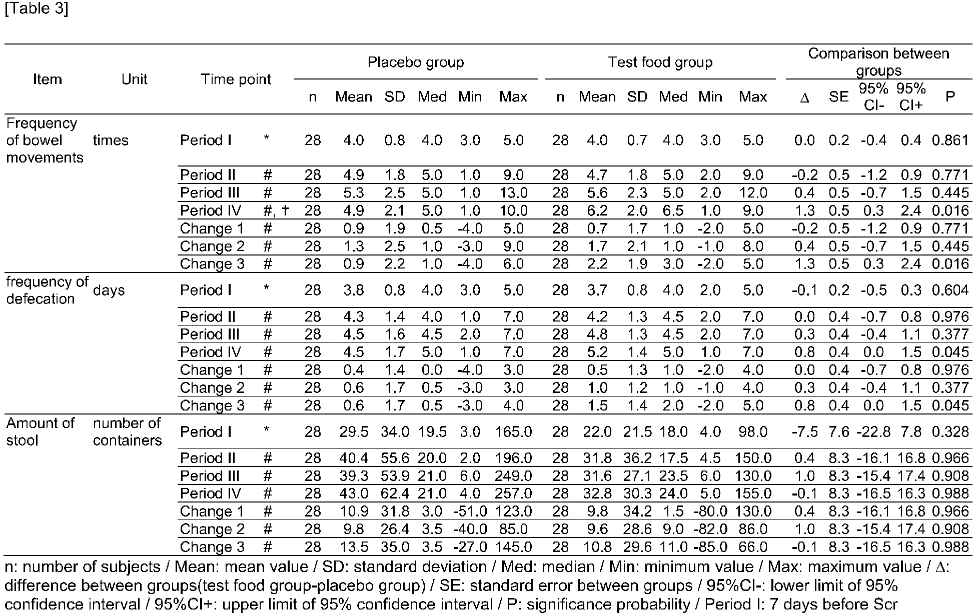

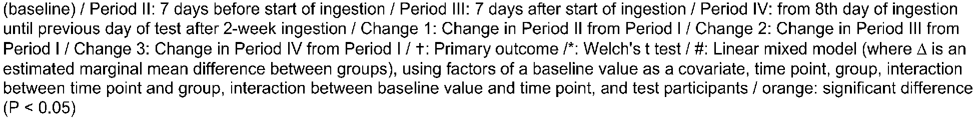

### 3.2. CAS-MT

### 3.2.1. Question Items

The Min, Q1, Med, Q3, Max, sum of ranks for each group, and the statistical analysis results for each question item were as follows. The following indicates, regarding items where a significant difference was observed, the time point at which the significant difference was observed, the Q1-Med-Q3, and the significance probability.

### (1) Low Frequency of Bowel Movements

Among time points where a significant difference was observed, a time point at which the test food group showed higher values than the placebo group was VISIT1 (placebo group: 0.0 - 0.5 - 1.0, test food group: 1.0 - 1.0 - 1.0, P = 0.011).

### (2) Small Amount of Stool

Among time points where a significant difference was observed, a time point at which the test food group showed higher values than the placebo group was VISIT1 (placebo group: 0.0 - 0.0 - 0.3, test food group: 0.0 - 1.0 - 1.0, P = 0.022).

From the above, it was found that the ingestion of the hydroxy fatty acid and/or the oxo fatty acid having 14 to 22 carbon atoms increased the frequency of bowel movements and the amount of stool.

### 3.3. Bacterial Occupancy Rate in Stool

The Mean and SD, Med, Min, Max, a difference between groups (Δ) and its SE, 95% CI-, 95% Cl+, and the statistical analysis results for the bacterial occupancy rate in stool were as follows. The following shows, regarding items where a significant difference was observed, the time point at which the significant difference was observed, the Mean and SD, the difference between groups, and 95% Cl- and 95% Cl+ thereof, and the significance probability. If, among the items where a significant difference was observed, a certain item was detected in one group but not detected in the other group at the baseline, the incident was documented.

### (1) d Bacteria; p Firmicutes; c Clostridia; o Lachnospirales; f Lachnospiraceae; g Agathobacter

Among time points where a significant difference was observed, a time point at which the test food group showed lower values than the placebo group was VISIT1 (placebo group: 0.0343 ± 0.0297, test food group: 0.0186 ± 0.0254, difference between groups: -0.0157 [-0.0305, -0.0009], P = 0.038).

### (2) d Bacteria; p Firmicutes; c Clostridia; o Oscillospirales; f Ruminococcaceae; g Ruminococcus

Among time points where a significant difference was observed, a time point at which the test food group showed lower values than the placebo group was VISIT1 (placebo group: 0.0134 ± 0.0147, test food group: 0.0037 ± 0.0075, difference between groups: -0.0097 [-0.0160, -0.0034], P = 0.003).

### (3) d Bacteria; p Firmicutes; c Clostridia; o Lachnospirales; f Lachnospiraceae; g [Ruminococcus] gauvreauii group

Among time points where a significant difference was observed, a time point at which the test food group showed lower values than the placebo group was VISIT1 (placebo group: 0.0054 ± 0.0069, test food group: 0.0020 ± 0.0046, difference between groups: -0.0034 [-0.0066, -0.0003], P = 0.034).

### (4) d Bacteria; p Proteobacteria; c Gammaproteobacteria; o Burkholderiales; f Sutterellaceae; g Sutterella

Among time points where a significant difference was observed, a time point at which the test food group showed higher values than the placebo group was VISIT1 (placebo group: 0.0034 ± 0.0038, test food group: 0.0082 ± 0.0086, difference between groups: 0.0048 [0.0012, 0.0084], P ≤ 0.010).

### (5) d Bacteria; p Firmicutes; c Bacilli; o Erysipelotrichales; f Erysipelotrichaceae; g Holdemanella

Among time points where a significant difference was observed, a time point at which the test food group showed lower values than the placebo group was VISIT1 (placebo group: 0.0069 ± 0.0141, test food group: 0.0007 ± 0.0028, difference between groups: -0.0062 [-0.0118, -0.0006], P = 0.030).

### (6) d Bacteria; p Firmicutes; c Bacilli; o Bacillales; f Bacillaceae; g Bacillus

Among time points where a significant difference was observed, a time point at which the test food group showed higher values than the placebo group was VISIT2 (placebo group: 0.0016 ± 0.0039, test food group: 0.0051 ± 0.0191, difference between groups: 0.0074 [0.0016, 0.0132], P = 0.014), and Change (placebo group: -0.0015 ± 0.0040, test food group: 0.0043 ± 0.0152, difference between groups: 0.0074 [0.0016, 0.0132], P = 0.014).

### (7) d Bacteria; p Firmicutes; c Clostridia; o Peptostreptococcales-Tissierellales; f Anaerovoracaceae

Among time points where a significant difference was observed, time points at which the test food group showed lower values than the placebo group was VISIT2 (placebo group: 0.0005 ± 0.0007, test food group: 0.0003 ± 0.0004, difference between groups: -0.0002 [-0.0005, 0.0000], P = 0.049), and Change (placebo group: 0.0001 ± 0.0005, test food group: -0.0001 ± 0.0005, difference between groups: - 0.0002 [-0.0005, 0.0000], P = 0.049).

### (8) d Bacteria; p Firmicutes; c Clostridia; o Lachnospirales; f Lachnospiraceae; g Hungatella

Among time points where a significant difference was observed, a time point at which the test food group showed lower values than the placebo group was VISIT1 (placebo group: 0.0003 ± 0.0006, test food group: 0.0001 ± 0.0001, difference between groups: -0.0002 [-0.0005, 0.0000], P = 0.049).

### (9) d Bacteria; p Firmicutes; c Clostridia; o Lachnospirales; f Lachnospiraceae; g Oribacterium

Among time points where a significant difference was observed, a time point at which the test food group showed lower values than the placebo group was Change (placebo group: -0.0003 ± 0.0015, test food group: 0.0000 ± 0.0000, difference between groups: 0.0000 [0.0000, 0.0000], P ≤ 0.001). At the baseline, the bacterium was detected only in the placebo group and not in the test food group.

### (10) d Bacteria; p Firmicutes; c Clostridia; o Peptostreptococcales-Tissierellales; f Peptostreptococcaceae; g Peptoclostridium

Among time points where a significant difference was observed, a time point at which the test food group showed higher values than the placebo group was VISIT2 (placebo group: 0.0000 ± 0.0000, test food group: 0.0002 ± 0.0013, difference between groups: 0.0000 [0.0000, 0.0000], P ≤ 0.001). At the baseline, the bacterium was detected only in the test food group and not in the placebo group.

### (11) d Bacteria; p Firmicutes; c Clostridia; o Lachnospirales; f Lachnospiraceae; g [Bacteroides] pectinophilus group

Among time points where a significant difference was observed, a time point at which the test food group showed lower values than the placebo group was VISIT2 (placebo group: 0.0000 ± 0.0001, test food group: 0.0000 ± 0.0000, difference between groups: 0.0000 [0.0000, 0.0000], P ≤ 0.001), and a time point at which the test food group showed higher values was Change (placebo group: - 0.0003 ± 0.0017, test food group: 0.0000 ± 0.0000, difference between groups: 0.0000 [0.0000, 0.0000], P ≤ 0.001). At the baseline, the bacterium was detected only in the placebo group and not in the test food group.

### (12) d Bacteria; p Proteobacteria; c Gammaproteobacteria; o Aeromonadales; f Succinivibrionaceae; g Anaerobiospirillum

Among time points where a significant difference was observed, a time point at which the test food group showed higher values than the placebo group was Change (placebo group: 0.0000 ± 0.0000, test food group: -0.0001 ± 0.0006, difference between groups: 0.0000 [0.0000, 0.0000], P ≤ 0.001). At the baseline, the bacterium was detected only in the test food group and not in the placebo group.

### (13) d Bacteria; p Firmicutes; c Clostridia; o Oscillospirales; f Ruminococcaceae; g Candidatus Soleaferrea

Among time points where a significant difference was observed, a time point at which the test food group showed lower values than the placebo group was VISIT1 (placebo group: 0.0001 ± 0.0001, test food group: 0.0000 ± 0.0000, difference between groups: 0.0000 [-0.0001, 0.0000], P ≤ 0.010).

### (14) d Bacteria; p Actinobacteriota; c Coriobacteriia; o Coriobacteriales; f Eggerthellaceae; guncultured

Among time points where a significant difference was observed, a time point at which the test food group showed higher values than the placebo group was Change (placebo group: 0.00001 ± 0.00005, test food group: 0.00000 ± 0.00000, difference between groups: 0.00000 [0.00000, 0.00000], P ≤ 0.001). At the baseline, the bacterium was detected only in the placebo group and not in the test food group.

### (15) d Bacteria; p Bacteroidota; c Bacteroidia; o Bacteroidales; f Prevotellaceae

Among time points where a significant difference was observed, a time point at which the test food group showed higher values than the placebo group was Change (placebo group: 0.00000 ± 0.00000, test food group: 0.00000 ± 0.00001, difference between groups: 0.00000 [0.00000, 0.00000], P ≤ 0.001). At the baseline, the bacterium was detected only in the test food group and not in the placebo group.

### (16) d Bacteria; p Firmicutes; c Clostridia; o Peptostreptococcales-Tissierellales; f Peptostreptococcaceae; g Tepidibacter

Among time points where a significant difference was observed, a time point at which the test food group showed higher values than the placebo group was VISIT2 (placebo group: 0.0000 ± 0.0001, test food group: 0.0000 ± 0.0000, difference between groups: 0.0000 [0.0000, 0.0000], P ≤ 0.001). At the baseline, the bacterium was detected only in the placebo group and not in the test food group.

### (17) d Bacteria; p Firmicutes; c Clostridia; o Lachnospirales; f Lachnospiraceae; g Anaerosporobacter

Among time points where a significant difference was observed, a time point at which the test food group showed higher values than the placebo group was VISIT2 (placebo group: 0.0000 ± 0.0002, test food group: 0.0000 ± 0.0000, difference between groups: 0.0000 [0.0000, 0.0000], P ≤ 0.001). At the baseline, the bacterium was detected only in the placebo group and not in the test food group.

### (18) d Bacteria; p Firmicutes; c Bacilli; o Erysipelotrichales; f Erysipelatoclostridiaceae

Among time points where a significant difference was observed, a time point at which the test food group showed lower values than the placebo group was Change (placebo group: 0.00000 ± 0.00001, test food group: 0.00000 ± 0.00000, difference between groups: 0.00000 [0.00000, 0.00000], P ≤ 0.001), and a time point at which the test food group showed higher values was VISIT2 (placebo group: 0.0000 ± 0.0001, test food group: 0.0000 ± 0.0000, difference between groups: 0.0000 [0.0000, 0.0000], P ≤ 0.001). At the baseline, the bacterium was detected only in the placebo group and not in the test food group.

### (19) d Bacteria; p Bacteroidota; c Bacteroidia; o Bacteroidales; f Dysgonomonadaceae; g Dysgonomonas

Among time points where a significant difference was observed, a time point at which the test food group showed lower values than the placebo group was VISIT2 (placebo group: 0.00001 ± 0.00003, test food group: 0.00000 ± 0.00000, difference between groups: 0.00000 [0.00000, 0.00000], P ≤ 0.001). At the baseline, the bacterium was detected only in the placebo group and not in the test food group.

### (20) d Bacteria; p Firmicutes; c Clostridia; ouncultured; funcultured; guncultured

Among time points where a significant difference was observed, a time point at which the test food group showed lower values than the placebo group was VISIT2 (placebo group: 0.0000 ± 0.0000, test food group: 0.0000 ± 0.0001, difference between groups: 0.0000 [0.0000, 0.0000], P ≤ 0.001), and a time point at which the test food group showed higher values was Change (placebo group: 0.00000 ± 0.00000, test food group: 0.00000 ± 0.00002, difference between groups: 0.00000 [0.00000, 0.00000], P ≤ 0.001). At the baseline, the bacterium was detected only in the test food group and not in the placebo group.

### (21) d Bacteria; p Proteobacteria; c Gammaproteobacteria; o Aeromonadales; f Succinivibrionaceae; g Succinivibrio

Among time points where a significant difference was observed, a time point at which the test food group showed lower values than the placebo group was VISIT2 (placebo group: 0.0000 ± 0.0000, test food group: 0.0000 ± 0.0001, difference between groups: 0.0000 [0.0000, 0.0000], P ≤ 0.001), and a time point at which the test food group showed higher values was Change (placebo group: 0.00000 ± 0.00000, test food group: 0.00001 ± 0.00003, difference between groups: 0.00000 [0.00000, 0.00000], P ≤ 0.001). At the baseline, the bacterium was detected only in the test food group and not in the placebo group.

### (22) d Archaea; p Euryarchaeota; c Methanobacteria; o Methanobacteriales; f Methanobacteriaceae; g Methanosphaera

Among time points where a significant difference was observed, time points at which the test food group showed higher values than the placebo group were VISIT2 (placebo group: 0.00001 ± 0.00004, test food group: 0.00000 ± 0.00000, difference between groups: 0.00000 [0.00000, 0.00000], P ≤ 0.001), and Change (placebo group: 0.00000 ± 0.00002, test food group: 0.00000 ± 0.00000, difference between groups: 0.00000 [0.00000, 0.00000], P ≤ 0.001). At the baseline, the bacterium was detected only in the placebo group and not in the test food group.

### (23) d Bacteria; p Firmicutes; c Clostridia; o Lachnospirales; f Lachnospiraceae; g Lachnospiraceae

Among time points where a significant difference was observed, a time point at which the test food group showed lower values than the placebo group was Change (placebo group: 0.0000 ± 0.0000, test food group: 0.0000 ± 0.0001, difference between groups: 0.0000 [0.0000, 0.0000], P ≤ 0.001). At the baseline, the bacterium was detected only in the test food group and not in the placebo group.

### (24) d Bacteria; p Actinobacteriota; c Actinobacteria; o Propionibacteriales; f Propionibacteriaceae; g Cutibacterium

Among time points where a significant difference was observed, a time point at which the test food group showed higher values than the placebo group was Change (placebo group: 0.00000 ± 0.00002, test food group: 0.00000 ± 0.00000, difference between groups: 0.00000 [0.00000, 0.00000], P ≤ 0.001). At the baseline, the bacterium was detected only in the placebo group and not in the test food group.

### (25) d Eukaryota

Among time points where a significant difference was observed, a time point at which the test food group showed higher values than the placebo group was Change (placebo group: 0.00000 ± 0.00000, test food group: 0.00000 ± 0.00001, difference between groups: 0.00000 [0.00000, 0.00000], P ≤ 0.001). At the baseline, the eukaryote was detected only in the test food group and not in the placebo group.

## Claims

1. An intestinal regulating agent comprising a hydroxy fatty acid and/or an oxo fatty acid having 14 to 22 carbon atoms.

2. The intestinal regulating agent according to claim 1, wherein the hydroxy fatty acid has a hydroxyl group at positions 10, 11, 12, 13, 14, and/or 15, and the oxo fatty acid has a carbonyl group at positions 10, 11, 12, 13, 14, and/or 15.

3. The intestinal regulating agent according to claim 1, wherein the hydroxy fatty acid or the oxo fatty acid is a saturated fatty acid, or an unsaturated fatty acid having one to five double bonds.

4. The intestinal regulating agent according to claim 1, wherein the hydroxy fatty acid is 10-hydroxy-cis-12-octadecenoic acid.

5. The intestinal regulating agent according to any one of claims 1 to 4, wherein the intestinal regulating agent is a food product or a food additive.

6. The intestinal regulating agent according to any one of claims 1 to 4, wherein the intestinal regulating agent is a medicament or a quasi-drug.

7. The intestinal regulating agent according to any one of claims 1 to 4, wherein the intestinal regulating agent is feed or feed additive.
